# EUROPÄISCHE PATENTSCHRIFT

(11) **EP 3 744 310 B1**
(45) Veröffentlichungstag und Bekanntmachung des Hinweises auf die Patenterteilung: **02.03.2022**
(21) Anmeldenummer: 20174079.2
(22) Anmeldetag: 12.05.2020
(51) Int. Cl.: A61K 8/37, A61K 8/49, A61Q 19/00

(54) **ZUSAMMENSETZUNGEN ENTHALTEND SORBITAN- UND GLYCERINCARBONSÄUREESTER**
COMPOSITIONS CONTAINING SORBITAN CARBOXYLIC ACID ESTERS AND GLYCERIN CARBOXYLIC ACID ESTERS
COMPOSITIONS CONTENANT DES ESTERS CARBONIQUES DE SORBITAN ET DES ESTERS CARBONIQUES DE GLYCÉRINE

(30) Priorität: 28.05.2019 EP 19176865
(43) Veröffentlichungstag der Anmeldung: 02.12.2020
(73) Patentinhaber: Evonik Operations GmbH, 45128 Essen (DE)
(72) Erfinder: Brandt, Kathrin Daniela, 40476 Düsseldorf (DE); Schuch, Dominik, 40221 Düsseldorf (DE); Bednorz, Beata, 45219 Essen (DE)
(74) Vertreter: Evonik Patent Association

(56) Entgegenhaltungen:
- WO-A1-2009/135007
- WO-A1-2012/062519
- WO-A2-2010/108738

## Beschreibung

### Gebiet der Erfindung

Gegenstand der Erfindung sind Zusammensetzungen enthaltend bestimmte Sorbitan- und Glycerincarbonsäureester in bestimmten Mengen.

### Stand der Technik

EP2410979 offenbart die Verwendung von Sorbitancarbonsäureestern, bei denen der Carbonsäureanteil sich ableitet von einer Carbonsäure enthaltend 6 bis 10 Kohlestoff-Atome und die eine Hydroxylzahl (OH-Zahl) von größer 350 aufweisen, als Viskositätsregler, Pflegewirkstoff, Schaum-Booster oder Solubilisator in reinigenden oder pflegenden Formulierungen.

Aufgabe der Erfindung war es, Zusammensetzungen bereitzustellen, welche über sehr gute Verdickungsleistungen verfügen.

### Beschreibung der Erfindung

Überraschenderweise wurde gefunden, dass die im Folgenden beschriebenen Zusammensetzungen enthaltend bestimmte Sorbitan- und Glycerincarbonsäureester die der Erfindung gestellte Aufgabe zu lösen vermögen.

Gegenstand der vorliegenden Erfindung sind daher Zusammensetzungen enthaltend Sorbitan- und Glycerincarbonsäureester sowie Wasser wie in Anspruch 1 beschrieben.

Ein weiterer Gegenstand der vorliegenden Erfindung ist ein Verfahren zur Herstellung von Formulierungen mit bestimmten Viskositäten unter Einsatz der erfindungsgemäßen Zusammensetzungen.

Noch ein Gegenstand der vorliegenden Erfindung ist die Verwendung der erfindungsgemäßen Zusammensetzungen zur Erhöhung der Viskosität einer kosmetischen oder pharmazeutischen Formulierung.

Ein Vorteil der erfindungsgemäßen Zusammensetzungen ist, dass sie eine bessere Verarbeitbarkeit aufweisen als die Einzelkomponenten.

Die erfindungsgemäßen Zusammensetzungen lassen sich bei gleichen Randbedingungen schneller homogen in kosmetische Formulierungen einarbeiten als die Einzelkomponenten.

Ein weiterer Vorteil der erfindungsgemäßen Zusammensetzungen ist, dass sie eine bessere Verdickungsleistung in kosmetischen Formulierungen zeigen als die Einzelkomponenten.

Ein weiterer Vorteil der erfindungsgemäßen Zusammensetzungen ist, dass sie bessere hautpflegende und sensorische Eigenschaften aufweisen als die Einzelkomponenten.

Gegenstand der vorliegenden Erfindung ist somit eine Zusammensetzung enthaltend
A) mindestens einen Sorbitancarbonsäureester mindestens einer Carbonsäure ausgewählt aus Carbonsäuren aufweisend 6 bis 12, bevorzugt 8 bis 10, Kohlenstoff-Atome, wobei alle in der Komponente A enthaltenen Sorbitancarbonsäureester zusammen im Mittel einen Veresterungsgrad von 0,7 bis 2,1, bevorzugt 0,9 bis 1,9, besonders bevorzugt 1,3 bis 1,7, Carbonsäurereste pro Sorbitancarbonsäureester aufweisen,
B) mindestens einen Glycerincarbonsäureester mindestens einer Carbonsäure ausgewählt aus Carbonsäuren aufweisend 6 bis 22, bevorzugt 12 bis 18, Kohlenstoff-Atome, wobei alle in der Komponente B enthaltenen Glycerincarbonsäureester zusammen im Mittel einen Veresterungsgrad von 0,7 bis 1,5, bevorzugt 1,0 bis 1,4, Carbonsäurereste pro Glycerincarbonsäureester aufweisen, und
C) Wasser,
dadurch gekennzeichnet, dass die Komponenten A) und B) in Summe bezogen auf die Gesamtzusammensetzung zu mindestens 50 Gew.-%, bevorzugt zu mindestens 65 Gew.-%, besonders bevorzugt zu mindestens 75 Gew.-%, insbesondere zu mindestens 85 Gew.-%, enthalten sind.

Alle angegebenen Prozent (%) sind, wenn nicht anders angegeben, Massenprozent. Sorbitanester sind als gute und milde Emulgatoren seit langem bekannt,

Sorbitol ist die reduzierte Polyolform der Glucose, zählt zu den Zuckeralkoholen und ist auch unter den Namen Sorbit oder Glucitol bekannt.

Sorbitol kann unter Abspaltung von Wasser eigenkondensieren, hierbei entsteht das sogenannte Sorbitan. Unter Sorbitan versteht man im Allgemeinen ein Produktgemisch der Eigenkondensationsprodukte des Sorbitols; diese sind im Wesentlichen fünf- und sechsgliederige, mono- und bicyclische, hydroxyfunktionelle Ether polyolischen Charakters, wie exemplarisch durch die folgenden Formeln dargestellt:

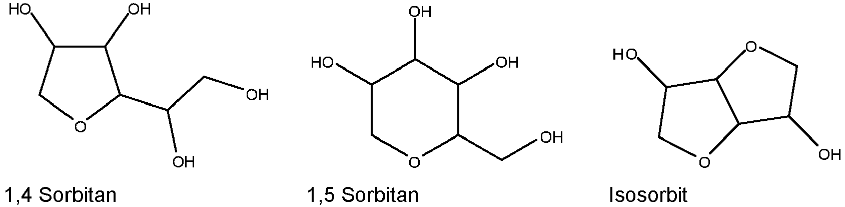

In diesem Gemisch sind im Allgemeinen im untergeordneten Ausmaß weitere Kondensationsprodukte und auch Sorbitol enthalten.

Sorbitancarbonsäureester sind die Carbonsäureester des Sorbitans und somit die Acylierungsprodukte dieses oben beschriebenen Polyolgemisches, wobei das Polyolgemisch in der Regel mit 1 bis 3 mol Carbonsäure pro 1 mol Polyol acyliert ist; es sind aber auch unterstöchiometrische Acylierungen denkbar, bei denen das Polyolgemisch mit weniger als 1 mol Carbonsäure pro 1 mol Polyol acyliert wird. Die im Zusammenhang mit der Erfindung für die Sorbitancarbonsäureester im Mittel angegebenen Veresterungsgrade beziehen sich auf das Stoffmengenverhältnis von Carbonsäure zu zur Eigenkondensation eingesetztem Sorbitol.

Eine Übersichtsdarstellung von Sorbitanestern findet sich beispielsweise in Treon, Soap Perfumary Cosmetics, January 1965, p. 47.

Die in der Komponente B enthaltenen Glycerincarbonsäureester können im Mittel einen Veresterungsgrad von kleiner 1 aufweisen, welches einem unterstöchiometrischem Acylierungsprodukt von Glycerin mit Carbonsäure entspricht. Solche Glycerincarbonsäureester umfassen somit Glycerin.

Es ist erfindungsgemäß bevorzugt, wenn die Komponente A) eine Verseifungszahl von 100 bis 300, bevorzugt 130 bis 280, besonders bevorzugt 160 bis 260 mg KOH / g aufweist.

Es ist erfindungsgemäß bevorzugt, wenn die Komponente B) eine Verseifungszahl von 100 bis 300, bevorzugt 115 bis 265, besonders bevorzugt 130 bis 230 mg KOH / g aufweist.

Geeignete Verfahren zur Bestimmung der Verseifungszahl der Komponenten A) und B) sind in DGF C-V 3, DIN EN ISO 3681 und Ph.Eur. 2.5.6 offenbart; diese ursprünglich für Fette entwickelte Verfahren lassen sich problemlos auf die Komponenten der Erfindung anwenden.

Eine erfindungsgemäß bevorzugte Zusammensetzung enthält
A) mindestens einen Sorbitancarbonsäureester mindestens einer Carbonsäure ausgewählt aus Carbonsäuren aufweisend 8 bis 10 Kohlenstoff-Atome, wobei alle in der Komponente A enthaltenen Sorbitancarbonsäureester zusammen im Mittel einen Veresterungsgrad von 1,3 bis 1,7, Carbonsäurereste pro Sorbitancarbonsäureester aufweisen,
B) mindestens einen Glycerincarbonsäureester mindestens einer Carbonsäure ausgewählt aus Carbonsäuren aufweisend 12 bis 18 Kohlenstoff-Atome, wobei alle in der Komponente B enthaltenen Glycerincarbonsäureester zusammen im Mittel einen Veresterungsgrad von 0,7 bis 1,5 Carbonsäurereste pro Glycerincarbonsäureester aufweisen, und
C) Wasser,
   und ist dadurch gekennzeichnet, dass die Komponenten A) und B) in Summe bezogen auf die Gesamtzusammensetzung zu mindestens 65 Gew.-%, bevorzugt zu mindestens 75 Gew.-%, besonders bevorzugt zu mindestens 85 Gew.-%, enthalten sind.

Eine erfindungsgemäß bevorzugte Zusammensetzung ist dadurch gekennzeichnet, dass sie 20 bis 75, bevorzugt 30 bis 65, besonders bevorzugt 40 bis 55, Gewichtsteile der Komponente A), 20 bis 75, bevorzugt 30 bis 65, besonders bevorzugt 40 bis 55, Gewichtsteile der Komponente B), und
0,01 bis 50, bevorzugt 0,1 bis 30, besonders bevorzugt 1 bis 10, Gewichtsteile Wasser, enthält.

Es ist erfindungsgemäß bevorzugt, dass in der erfindungsgemäßen Zusammensetzung Komponente B) mindestens 70, bevorzugt mindestens 80 Gew.-% Glycerinmonocarbonsäureester enthält, wobei sich die Gewichtsprozente auf alle Glycerincarbonsäureesterder Komponente B) beziehen.

Eine erfindungsgemäß bevorzugte Zusammensetzung ist dadurch gekennzeichnet, dass die Carbonsäuren der Carbonsäureester der Komponenten A) und B) ausgewählt sind aus Fettsäuren.

Es ist erfindungsgemäß bevorzugt, dass in der erfindungsgemäßen Zusammensetzung die Carbonsäure des Sorbitancarbonsäureesters der Komponente A) ausgewählt ist aus Caprylsäure und Caprinsäure. Eine besonders bevorzugte erfindungsgemäße Zusammensetzung ist dadurch gekennzeichnet, dass die Carbonsäure des Sorbitancarbonsäureesters der Komponente A) eine Mischung aus Caprylsäure und Caprinsäure darstellt, bevorzugt mit einem Gewichtsverhältnis von Caprylsäure zu Caprinsäure in einem Bereich von 6 zu 1 bis 2 zu 1, bevorzugt 4 zu 1 bis 3 zu 1.

Es ist erfindungsgemäß bevorzugt, dass in der erfindungsgemäßen Zusammensetzung die Carbonsäure des Glycerincarbonsäureesters der Komponente B) ausgewählt ist aus Laurinsäure und Ölsäure, bevorzugt Ölsäure.

Eine erfindungsgemäß bevorzugte Zusammensetzung ist dadurch gekennzeichnet, dass sie eine Viskosität von 50 bis 5000, bevorzugt 100 bis 3000 mPa s, gemessen bei 25 °C mit einem Brookfield-Viskosimeter mit Spindel 62 und bei 30 rpm, aufweist.

Die erfindungsgemäßen Zusammensetzungen stellen Konzentrate dar, mit denen sich in vorteilhafter Art und Weise Formulierungen herstellen lassen, bei denen die Wirkstoffkonzentration im üblichen Anwendungsbereich liegt.

Somit ist ein weiterer Gegenstand der vorliegenden Erfindung ein Verfahren zur Herstellung einer kosmetischen oder pharmazeutischen Formulierung, insbesondere mit einer Viskosität von 500 bis 15000 mPa s, bevorzugt 1000 bis 10000 mPa s, gemessen bei 25 °C mit einem Brookfield-Viskosimeter mit Spindel 62 und bei 30 rpm, umfassend die Verfahrensschritte
I) Bereitstellen einer erfindungsgemäßen Zusammensetzung,
II) Vermengen der erfindungsgemäßen Zusammensetzung mit weiteren kosmetischen oder pharmazeutischen Formulierungsbestandteilen, unter Erhalt einer Formulierung mit einem Gehalt von Komponente A) und Komponente B) in Summe in einem Bereich von 0,1 Gew.-% bis 5,0 Gew.-%, bevorzugt 0,2 Gew.-% bis 2,5 Gew.-%, wobei sich die Gewichtsprozente auf die Gesamtformulierung beziehen.

In dem erfindungsgemäßen Verfahren zur Herstellung einer kosmetischen oder pharmazeutischen Formulierung kommen als in Verfahrensschritt II) eingesetzte Formulierungsbestandteile beispielsweise in Betracht:
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

In dem erfindungsgemäßen Verfahren zur Herstellung einer kosmetischen oder pharmazeutischen Formulierung wird als in Verfahrensschritt II) eingesetzter Formulierungsbestandteil insbesondere mindestens ein Tensid umfasst.

Die Carbonsäureester der erfindungsgemäßen Zusammensetzung können tensidische Eigenschaften aufweisen; im Zusammenhang mit der vorliegenden Erfindung werden diese Carbonsäureester nicht zu den Tensiden mitgezählt.

Umfasste Tenside können beispielsweise anionische, nichtionische oder amphotere Tenside sein. Typische Beispiele für anionische Tenside sind Fettalkoholsulfate,

Fettalkoholpolyglycolethersulfate, Monoglyceridsulfate, Mono- und/oder Dialkylsulfosuccinate, Fettsäureisethionate, Fettsäuresarcosinate, Fettsäuretaurate, Fettsäureglutamate, Fettsäureglycinate, Alkylethercarboxylate, .

Nichtionische Tenside sind z.B. Alkyloligoglucoside, Fettsäureglucamide, Rhamnolipide, Sophorolipide und/oder Proteinfettsäurekondensate, letztere beispielsweise auf Basis von Weizenproteinen.

Amphotere Tenside sind z.B. Alkylamidoalkylhydroxysultaine, Alkylamidoalkylbetaine, Alkylbetaine, Amphoacetate und Amphopropionate, deren endständige Acyl- oder Alkylreste typischerweise 8 bis 18 Kohlenstoffatome enthalten.

Erfindungsgemäß insbesondere umfasste Tenside sind Fettalkoholsulfate, Fettalkoholpolyglycolethersulfate, Mono- und/oder Dialkylsulfosuccinate, Amphoacetate, Amphopropionate, Alkylbetaine, Cocamidopropylbetaine, Alkyloligoglucoside und Fettsäureglutamate.

Erfindungsgemäß besonders bevorzugt umfasste Tenside sind die polyetherfreien Tenside Mono- und/oder Dialkylsulfosuccinate, Amphoacetate, Amphopropionate, Betaine, insbesondere Cocamidopropylbetaine, Alkyloligoglucoside und Fettsäureglutamate.

Erfindungsgemäß bevorzugt wird die Menge des umfassten Tensides so eingesetzt, das die erhaltene Formulierung mindestens 2 Gew.-%, bevorzugt mindestens 4 Gew.-% und besonders bevorzugt mindestens 6 Gew.-%, Gesamttensid bezogen auf die Gesamtformulierung aufweist.

Ein erfindungsgemäß bevorzugtes Verfahren ist dadurch gekennzeichnet, dass Verfahrensschritt II) in einem Temperaturbereich von 15 °C bis 90 °C, bevorzugt von 18 °C bis 60 °C, durchgeführt wird.

Es ist erfindungsgemäß bevorzugt, dass in dem erfindungsgemäßen Verfahren in Verfahrensschritt II) ein Wassergehalt in einem Bereich von 50 Gew.-% bis 95 Gew.-%, bevorzugt 80 Gew.-% bis 93 Gew.-%, wobei sich die Gewichtsprozente auf die Gesamtformulierung beziehen, eingestellt wird.

Ein erfindungsgemäß bevorzugtes Verfahren ist dadurch gekennzeichnet, dass es Verfahrensschritt III) Einstellen des pH-Wertes auf einen Bereich von 4,0 bis 8,0, bevorzugt 4,5 bis 6,5, umfasst.

Der "pH-Wert" im Zusammenhang mit der vorliegenden Erfindung ist definiert als der Wert, welcher für die entsprechende Zusammensetzung bei 25 °C nach fünf Minuten Rühren mit einer gemäß ISO 4319 (1977) kalibrierten pH-Elektrode gemessen wird.

Ein weiterer Gegenstand der vorliegenden Erfindung ist die Verwendung einer erfindungsgemäßen Zusammensetzung zur Erhöhung der Viskosität einer kosmetischen oder pharmazeutischen, bevorzugt tensidischen, Formulierung. Da die Carbonsäureester der erfindungsgemäß verwendeten Zusammensetzung tensidische Eigenschaften aufweisen können, ist im Zusammenhang mit der vorliegenden Erfindung unter dem Begriff "tensidische Formulierung" eine Formulierung zu verstehen, die neben den vorgenannten Carbonsäureestern mindestens ein weiteres Tensid enthält.

Es ist offenbar, dass in bevorzugten erfindungsgemäßen Verfahren in Verfahrensschritt I) oben die als bevorzugt ausgewiesenen erfindungsgemäßen Zusammensetzungen eingesetzt werden. Analoges gilt für bevorzugte erfindungsgemäße Verwendungen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung einer erfindungsgemäßen Zusammensetzung

47.5 g Sorbitansesquicaprylat und 47.5 g Glycerylmonooleat und 5 g Wasser wurden bei 60°C für 30 min gerührt und anschließend auf 22°C abgekühlt.

### Beispiel 2: Einarbeitung in eine kosmetische Formulierung

Die Formulierungsbestandteile in den folgenden Zusammensetzungen sind in Form der allgemein anerkannten INCI-Nomenklatur unter Verwendung der englischen Begriffe benannt. Alle Konzentrationen in den Anwendungsbeispielen sind in Gew.-% angegeben.

Es wurde eine tensidische Formulierung bestehend aus 5,6% Sodium Cocoamphoacetate, 4,4% Lauryl Glucoside, 1,2% Coco-Glucoside und 3,6% Sod./Disod. Cocoyl Glutamate in Wasser hergestellt. Der pH-Wert wurde mit Citronensäure auf 5,5 eingestellt. Die Zeit, die benötigt wurde, um 1,0% Verdickungsmittel bei 22°C und 30 rpm (Ankerrührer) klar und homogen in diese Formulierung einzuarbeiten, wurde bestimmt und ist in folgender Tabelle dargestellt:

**Tabelle 1: Einarbeitungszeit von 1,0% Verdickungsmittel in die Beispielformulierung (22°C, Ankerrührer, 30 rpm)**

| | Einarbeitungszeit [min] |
|---|---|
| Beispiel 1 (erfindungsgemäß **) | 2 |
| Sorbitansesquicaprylat (nicht erfindungsgemäß *) | 5 |
| Glycerylmonooleat (nicht erfindungsgemäß *) | nach 100 min keine klare, homogene Formulierung |

Die Ergebnisse aus Tabelle 1 zeigen, dass für die klare und homogene Einarbeitung des erfindungsgemäßen Beispiels 1 weniger Zeit benötigt wird als für die nicht-erfindungsgemäßen Verdickungsmittel.

### Beispiel 2b: Einarbeitung in eine kosmetische Formulierung

Es wurde eine tensidische Formulierung bestehend aus 4,8% Sodium Cocoamphoacetate, 4,8% Cocamidopropyl Betaine und 3,6% Disodium Lauryl Sulfosuccinate in Wasser hergestellt. Der pH-Wert wurde mit Citronensäure auf 5,5 eingestellt. Die Zeit, die benötigt wurde, um 1,8% Verdickungsmittel bei 22°C und 250 rpm (Ankerrührer) klar und homogen in diese Formulierung einzuarbeiten, wurde bestimmt und ist in folgender Tabelle dargestellt:

**Tabelle 1b: Einarbeitungszeit von 1,8% Verdickungsmittel in die Beispielformulierung (22°C, Ankerrührer, 250 rpm)**

| | Einarbeitungszeit [min] |
|---|---|
| Beispiel 1 (erfindungsgemäß **) | 5 |
| Sorbitansesquicaprylat (nicht erfindungsgemäß *) | 6 |
| Glycerylmonooleat (nicht erfindungsgemäß *) | nach 200 min keine klare, homogene Formulierung |

Die Ergebnisse aus Tabelle 1b zeigen, dass für die klare und homogene Einarbeitung des erfindungsgemäßen Beispiels 1 weniger Zeit benötigt wird als für die nicht-erfindungsgemäßen Verdickungsmittel.

### Beispiel 3: Verdickungsleistung in einer kosmetischen Formulierung

Die verdickende Wirkung des erfindungsgemäßen Beispiels 1 wurde im Vergleich zu nicht-erfindungsgemäßen Verdickungsmitteln evaluiert. Hierfür wurde eine kosmetische Formulierung bestehend aus 6,8% Cocamidopropyl Betaine, 5,9% Lauryl Glucoside, 2,8 % Coco-Glucoside und 1,5% Sucrose Cocoate in Wasser hergestellt. Der pH-Wert dieser Formulierung wurde mit Citroonensäure auf 5,5 eingestellt. In diese Formulierungen wurden jeweils 0,5% Verdickungsmittel eingearbeitet und die Viskositäten mithilfe eines Brookfield-Viskosimeters (Spindel 62, 30 rpm Umdrehungen) bei 22 °C gemessen. Die Ergebnisse der Viskositätsmessungen sind in Tabelle 2 dargestellt:

**Tabelle 2: Viskosität der Beispielformulierung mit 0,5% Verdickungsmittel**

| | Viskosität [mPa s] |
|---|---|
| Beispiel 1 (erfindungsgemäß **) | 2800 |
| Sorbitansesquicaprylat (nicht erfindungsgemäß *) | 2170 |
| Glycerylmonooleat (nicht erfindungsgemäß *) | 600 |

Die Ergebnisse aus Tabelle 2 zeigen, dass mit dem erfindungsgemäßen Beispiel 1 höher viskose Formulierungen erhalten werden als mit den nicht-erfindungsgemäßen Beispielen.'

### Beispiel 4: Hautpflegevermögen

Die verbesserte Hautpflegeleistung des erfindungsgemäßen Beispiels 1 wurde im Vergleich zu nicht-erfindungsgemäßen Beispielen mithilfe eines sensorischen Handwaschtests evaluiert.

Hierfür wusch sich eine Gruppe bestehend aus 4 trainierten Prüfpersonen definiert die Hände und bewertete die Hautweichheit nach dem Waschen anhand einer Notenskala von 1 (sehr schlecht) bis 5 (sehr gut). Das erfindungsgemäße Beispiel 1 sowie die nicht-erfindungsgemäßen Beispiele wurden jeweils in einer standardisierten Tensidformulierung getestet, bestehend aus 9% aktiv Sodium Laureth Sulfate und 3% aktiv Cocamidopropyl Betaine (siehe Tabelle 3).

**Tabelle 3: Testformulierungen für den Handwaschtest, pH 5,5, Angaben in % Aktivsubstanz**

| **Formulierungsbeispiele** | **I **** | **II*** | **III*** |
|---|---|---|---|
| Texapon^{®} NSO-IS, BASF Cognis, 28%-ig, (INCI: Sodium Laureth Sulfate) | 9,0% | 9,0% | 9,0% |
| TEGO^{®} Betain F 50, Evonik Industries AG, 38%-ig, (INCI: Cocamidopropyl Betaine) | 3,0% | 3,0% | 3,0% |
| Sodium Chloride | 0,7% | 0,7% | 0,7% |
| Zitronensäure | q.s. | q.s. | q.s. |
| Wasser, demineralisiert | ad 100 | ad 100 | ad 100 |
| Beispiel 1 (erfindungsgemäß **) | 1,1% | | |
| Sorbitansesquicaprylat (nicht erfindungsgemäß *) | | 1,1% | |
| Glycerylmonooleat (nicht erfindungsgemäß *) | | | 1,1% |

Die sensorischen Testergebnisse sind in Tabelle 4 zusammengefasst.

**Tab. 4: Ergebnisse des Handwaschtests, Mittelwerte über alle Testpersonen**

| | **Formulierung I **** | **Formulierung II *** | **Formulierung III *** |
|---|---|---|---|
| Hautweichheit | **2,50** | 2,25 | 2,25 |
| Hautweichheit nach 3 min. | **3,50** | 3,25 | 3,13 |

Anhand der Testergebnisse in Tabelle 4 wird ersichtlich, dass Formulierung I unter Verwendung des erfindungsgemäßen Beispiels 1 zu einer Erhöhung der Hautweichheit und damit zu einem insgesamt besseren Hautgefühl führt.

## Patentansprüche

1. Zusammensetzung enthaltend
A) mindestens einen Sorbitancarbonsäureester mindestens einer Carbonsäure ausgewählt aus Carbonsäuren aufweisend 6 bis 12, bevorzugt 8 bis 10, Kohlenstoff-Atome, wobei alle in der Komponente A enthaltenen Sorbitancarbonsäureester zusammen im Mittel einen Veresterungsgrad von 0,7 bis 2,1, bevorzugt 0,9 bis 1,9, besonders bevorzugt 1,3 bis 1,7, Carbonsäurereste pro Sorbitancarbonsäureester aufweisen,
B) mindestens einen Glycerincarbonsäureester mindestens einer Carbonsäure ausgewählt aus Carbonsäuren aufweisend 6 bis 22, bevorzugt 12 bis 18, Kohlenstoff-Atome, wobei alle in der Komponente B enthaltenen Glycerincarbonsäureester zusammen im Mittel einen Veresterungsgrad von 0,7 bis 1,5 Carbonsäurereste pro Glycerincarbonsäureester aufweisen, und
C) Wasser,
**dadurch gekennzeichnet, dass** die Komponenten A) und B) in Summe bezogen auf die Gesamtzusammensetzung zu mindestens 50 Gew.-%, bevorzugt zu mindestens 65 Gew.-%, besonders bevorzugt zu mindestens 75 Gew.-%, insbesondere zu mindestens 85 Gew.-%, enthalten sind.

2. Zusammensetzung gemäß Anspruch 1, **dadurch gekennzeichnet, dass** die Komponente A) eine Verseifungszahl von 100 bis 300, bevorzugt 130 bis 280, besonders bevorzugt 160 bis 260 mg KOH / g aufweist.

3. Zusammensetzung gemäß Anspruch 1 oder 2, **dadurch gekennzeichnet, dass** die Komponente B) eine Verseifungszahl von 100 bis 300, bevorzugt 115 bis 265, besonders bevorzugt 130 bis 230 mg KOH / g aufweist.

4. Zusammensetzung gemäß mindestens einem der vorherigen Ansprüche, **dadurch gekennzeichnet, dass** sie
20 bis 75, bevorzugt 30 bis 65, besonders bevorzugt 40 bis 55, Gewichtsteile der Komponente A),
20 bis 75, bevorzugt 30 bis 65, besonders bevorzugt 40 bis 55, Gewichtsteile der Komponente B), und
0,01 bis 50, bevorzugt 0,1 bis 30, besonders bevorzugt 1 bis 10, Gewichtsteile Wasser, enthält.

5. Zusammensetzung gemäß mindestens einem der vorherigen Ansprüche, **dadurch gekennzeichnet, dass** Komponente B) mindestens 70, bevorzugt mindestens 80 Gew.-% Glycerinmonocarbonsäureester enthält, wobei sich die Gewichtsprozente auf alle Glycerincarbonsäureester der Komponente B) beziehen.

6. Zusammensetzung gemäß mindestens einem der vorherigen Ansprüche, **dadurch gekennzeichnet, dass** die Carbonsäuren der Carbonsäureester der Komponenten A) und B) ausgewählt sind aus Fettsäuren.

7. Zusammensetzung gemäß mindestens einem der vorherigen Ansprüche, **dadurch gekennzeichnet, dass** die Carbonsäure des Sorbitancarbonsäureesters der Komponente A) ausgewählt ist aus Caprylsäure und Caprinsäure, bevorzugt eine Mischung aus Caprylsäure und Caprinsäure, besonders bevorzugt mit einem Gewichtsverhältnis von Caprylsäure zu Caprinsäure in einem Bereich von 6 zu 1 bis 2 zu 1.

8. Zusammensetzung gemäß mindestens einem der vorherigen Ansprüche, **dadurch gekennzeichnet, dass** die Carbonsäure des Glycerincarbonsäureesters der Komponente B) ausgewählt ist aus ungesättigten oder verzweigten Fettsäuren, insbesondere Ölsäure.

9. Verfahren zur Herstellung einer kosmetischen oder pharmazeutischen Formulierung, insbesondere mit einer Viskosität von 500 bis 15000 mPa s, bevorzugt 1000 bis 10000 mPa s, gemessen bei 25 °C mit einem Brookfield-Viskosimeter mit Spindel 62 und bei 30 rpm, umfassend die Verfahrensschritte
I) Bereitstellen einer Zusammensetzung gemäß mindestens einem der vorhereigen Ansprüche,
II) Vermengen der Zusammensetzung mit weiteren kosmetischen oder pharmazeutischen Formulierungsbestandteilen, unter Erhalt einer Formulierung mit einem Gehalt von Komponente A) und Komponente B) in Summe in einem Bereich von 0,1 Gew.-% bis 5,0 Gew.-%, bevorzugt 0,2 Gew.-% bis 2,5 Gew.-%, wobei sich die Gewichtsprozente auf die Gesamtformulierung beziehen.

10. Verfahren gemäß Anspruch 9, **dadurch gekennzeichnet, dass** in Verfahrensschritt II) ein Wassergehalt in einem Bereich von 50 Gew.-% bis 95 Gew.-%, bevorzugt 80 Gew.-% bis 93 Gew.-%, wobei sich die Gewichtsprozente auf die Gesamtformulierung beziehen, eingestellt wird.

11. Verfahren gemäß Anspruch 9 oder 10 umfassend Verfahrensschritt III) Einstellen des pH-Wertes auf einen Bereich von 4,0 bis 8,0, bevorzugt 4,5 bis 6,5.

12. Verwendung einer Zusammensetzung gemäß mindestens einem der Ansprüch1 bis 8 zur Erhöhung der Viskosität einer kosmetischen oder pharmazeutischen Formulierung.

## Claims

1. Composition comprising
A) at least one sorbitan carboxylic ester of at least one carboxylic acid selected from carboxylic acids having 6 to 12, preferably 8 to 10, carbon atoms, wherein all sorbitan carboxylic esters present in component A together have on average a degree of esterification of 0.7 to 2.1, preferably 0.9 to 1.9, particularly preferably 1.3 to 1.7, carboxylic acid radicals per sorbitan carboxylic ester,
B) at least one glycerol carboxylic ester of at least one carboxylic acid selected from carboxylic acids having 6 to 22, preferably 12 to 18, carbon atoms, wherein all glycerol carboxylic esters present in component B together have on average a degree of esterification of 0.7 to 1.5 carboxylic acid radicals per glycerol carboxylic ester, and
C) water,
**characterized in that** components A) and B) in sum total are present to an extent of at least 50% by weight, preferably to an extent of at least 65% by weight, particularly preferably to an extent of at least 75% by weight, especially to an extent of at least 85% by weight, based on the total composition.

2. Composition according to Claim 1, **characterized in that** component A) has a saponification number of 100 to 300, preferably 130 to 280, particularly preferably 160 to 260 mg KOH/g.

3. Composition according to Claim 1 or 2, **characterized in that** component B) has a saponification number of 100 to 300, preferably 115 to 265, particularly preferably 130 to 230 mg KOH/g.

4. Composition according to at least one of the preceding claims, **characterized in that** said composition comprises
20 to 75, preferably 30 to 65, particularly preferably 40 to 55, parts by weight of component A),
20 to 75, preferably 30 to 65, particularly preferably 40 to 55, parts by weight of component B), and
0.01 to 50, preferably 0.1 to 30, particularly preferably 1 to 10, parts by weight of water.

5. Composition according to at least one of the preceding claims, **characterized in that** component B) comprises at least 70, preferably at least 80% by weight glycerol monocarboxylic ester, wherein the percentages by weight refer to all glycerol carboxylic esters of component B).

6. Composition according to at least one of the preceding claims, **characterized in that** the carboxylic acids of the carboxylic esters of components A) and B) are selected from fatty acids.

7. Composition according to at least one of the preceding claims, **characterized in that** the carboxylic acid of the sorbitan carboxylic ester of component A) is selected from caprylic acid and capric acid, preferably a mixture of caprylic acid and capric acid, particularly preferably with a ratio by weight of caprylic acid to capric acid in a range from 6:1 to 2:1.

8. Composition according to at least one of the preceding claims, **characterized in that** the carboxylic acid of the glycerol carboxylic ester of component B) is selected from unsaturated or branched fatty acids, especially oleic acid.

9. Process for preparing a cosmetic or pharmaceutical formulation, in particular having a viscosity of 500 to 15 000 mPa s, preferably 1000 to 10 000 mPa s, measured at 25°C with a Brookfield viscometer using spindle 62 and at 30 rpm, comprising the process steps of
I) providing a composition according to at least one of the preceding claims,
II) mixing the composition with further cosmetic or pharmaceutical formulation constituents to obtain a formulation having a content of component A) and component B) in sum total in a range of 0.1% by weight to 5.0% by weight, preferably 0.2% by weight to 2.5% by weight, wherein the percentages by weight refer to the total formulation.

10. Process according to Claim 9, **characterized in that** in process step II) a water content is set in a range of 50% by weight to 95% by weight, preferably 80% by weight to 93% by weight, wherein the percentages by weight refer to the total formulation.

11. Process according to Claim 9 or 10 comprising the process step of
III) adjusting the pH to a range of 4.0 to 8.0, preferably 4.5 to 6.5.

12. Use of a composition according to at least one of Claims 1 to 8 for increasing the viscosity of a cosmetic or pharmaceutical formulation.

## Revendications

1. Composition contenant
A) au moins un ester d'acide sorbitanecarboxylique d'au moins un acide carboxylique choisi parmi les acides carboxyliques présentant 6 à 12, de préférence 8 à 10 atomes de carbone, tous les esters d'acide sorbitanecarboxylique contenus dans le composant A présentant ensemble, en moyenne, un degré d'estérification de 0,7 à 2,1, de préférence de 0,9 à 1,9, de manière particulièrement préférée de 1,3 à 1,7, radicaux d'acide carboxylique par ester d'acide sorbitanecarboxylique,
B) au moins un ester d'acide glycérolcarboxylique d'au moins un acide carboxylique choisi parmi les acides carboxyliques présentant 6 à 22, de préférence 12 à 18 atomes de carbone, tous les esters d'acide glycérolcarboxylique contenus dans le composant B présentant ensemble, en moyenne, un degré d'estérification de 0,7 à 1,5 radical d'acide carboxylique par ester d'acide glycérolcarboxylique, et
C) de l'eau,
**caractérisée en ce que** les composants A) et B) sont contenus, au total, par rapport à la composition totale, à raison d'au moins 50% en poids, de préférence d'au moins 65% en poids, de manière particulièrement préférée d'au moins 75% en poids, en particulier d'au moins 85% en poids.

2. Composition selon la revendication 1, **caractérisée en ce que** le composant A) présente un indice de saponification de 100 à 300, de préférence de 130 à 280, de manière particulièrement préférée de 160 à 260 mg de KOH/g.

3. Composition selon la revendication 1 ou 2, **caractérisée en ce que** le composant B) présente un indice de saponification de 100 à 300, de préférence de 115 à 265, de manière particulièrement préférée de 130 à 230 mg de KOH/g.

4. Composition selon au moins l'une quelconque des revendications précédentes, **caractérisée en ce qu'**elle contient
- 20 à 75, de préférence 30 à 65, de manière particulièrement préférée 40 à 55 parties en poids du composant A),
- 20 à 75, de préférence 30 à 65, de manière particulièrement préférée 40 à 55 parties en poids du composant B) et
- 0,01 à 50, de préférence 0,1 à 30, de manière particulièrement préférée 1 à 10 parties en poids d'eau.

5. Composition selon au moins l'une quelconque des revendications précédentes, **caractérisée en ce que** le composant B) contient au moins 70, de préférence au moins 80% en poids d'ester d'acide glycérolmonocarboxylique, les % en poids se rapportant à tous les esters d'acide glycérolcarboxylique du composant B).

6. Composition selon au moins l'une quelconque des revendications précédentes, **caractérisée en ce que** les acides carboxyliques des esters d'acide carboxylique des composants A) et B) sont choisis parmi les acides gras.

7. Composition selon au moins l'une quelconque des revendications précédentes, **caractérisée en ce que** l'acide carboxylique de l'ester d'acide sorbitanecarboxylique du composant A) est choisi parmi l'acide caprylique et l'acide caprique, de préférence un mélange d'acide caprylique et d'acide caprique, de manière particulièrement préférée à un rapport pondéral d'acide caprylique à acide caprique dans une plage de 6:1 à 2:1.

8. Composition selon au moins l'une quelconque des revendications précédentes, **caractérisée en ce que** l'acide carboxylique de l'ester d'acide glycérolcarboxylique du composant B) est choisi parmi les acides gras insaturés ou ramifiés, en particulier l'acide oléique.

9. Procédé pour la préparation d'une formulation cosmétique ou pharmaceutique, présentant en particulier une viscosité de 500 à 15.000 mPa.s, de préférence de 1000 à 10.000 mPa.s, mesurée à 25°C à l'aide d'un viscosimètre Brookfield pourvu d'un mobile 62 et à 30 tr/min, comprenant les étapes de procédé
I) préparation d'une composition selon au moins l'une quelconque des revendications précédentes,
II) mélange de la composition avec d'autres constituants de formulation cosmétique ou pharmaceutique, avec obtention d'une formulation présentant une teneur en composant A) et en composant B), au total, dans une plage de 0,1% en poids à 5,0% en poids, de préférence de 0,2% en poids à 2,5% en poids, les % en poids se rapportant à la formulation totale.

10. Procédé selon la revendication 9, **caractérisé en ce qu'**on règle, dans l'étape de procédé II), une teneur en eau dans la plage de 50% en poids à 95% en poids, de préférence de 80% en poids à 93% en poids, les % en poids se rapportant à la formulation totale.

11. Procédé selon la revendication 9 ou 10, comprenant l'étape de procédé
III) réglage du pH à une plage de 4,0 à 8,0, de préférence de 4,5 à 6,5.

12. Utilisation d'une composition selon au moins l'une quelconque des revendications 1 à 8 pour augmenter la viscosité d'une formulation cosmétique ou pharmaceutique.
